# EUROPEAN PATENT APPLICATION

(11) **EP 4 604 136 A1**
(43) Date of publication of application: **20.08.2025**
(21) Application number: 25156149.4
(22) Date of filing: 06.02.2025
(51) Int. Cl.: G16H 80/00, A61B 5/00

(54) **ELECTRONIC APPARATUS AND CONTROLLING METHOD THEREOF**

(30) Priority: 13.02.2024 KR 20240020601
(71) Applicant: I-SENS, INC., Seoul 06646 (KR)
(72) Inventor: Jang, Ho Jun, 06646 Seoul (KR); Park, Kyong Lyol, 06646 Seoul (KR); Chun, Lee Seung, 06646 Seoul (KR)
(74) Representative: Westphal, Mussgnug & Partner Patentanwälte mbB

(57) **Abstract**

A method performed by an electronic apparatus is disclosed. The method comprises receiving information about a user's glucose concentration from an analyte monitoring device attached to the user's body, receiving an alert condition set by a follower of the user at the follower's device, determining whether the alert condition is satisfied based on the information about the user's glucose concentration, and controlling the follower's device to output an alert when the alert condition is satisfied.

## Description

### CROSS-REFERENCE TO RELATED APPLICATIONS

This application claims priority from Korean Patent Application No. 10-2024-0020601 filed in the Korean Intellectual Property Office on February 13, 2024, the disclosures of which are incorporated herein by reference.

### BACKGROUND

### 1. Technical Field

The present disclosure relates to an analyte monitoring device, and more particularly, to a continuous glucose monitoring (CGM) device and a controlling method thereof.

### 2. Related Art

Continuous glucose monitoring systems (CGMSs) are systems that acquire blood sugar concentrations of users using sensors in contact with body fluids (e.g., interstitial fluid) of the users and provide the acquired blood sugar concentrations to the users. A CGM system includes CGM device attached to the body of a user to sense signals from the body fluid of the user, and a user terminal device that provides the blood sugar concentration to the user.

The user terminal notifies the user by outputting an alert when there is an abnormality in the blood glucose value of a continuous glucose monitor user. However, if the user does not immediately recognize this, they may be at risk, so it is necessary to also provide notifications to the user's followers (such as guardians, caregivers, or medical personnel). Therefore, technology is needed to provide notifications about the user's blood glucose status to followers.

### SUMMARY

The present disclosure provides an electronic apparatus that provides alerts about glucose concentration of a user of an analyte monitoring device to followers.

The present disclosure provides an electronic apparatus that provides alerts based on alert conditions set by followers.

Objects of the present disclosure are not limited to the above-mentioned objects. That is, other objects that are not described may be obviously understood by those skilled in the art to which the present disclosure pertains from the following description.

According to an embodiment of the present disclosure, there is provided a method of controlling an electronic apparatus, the method comprising: receiving information about a user's glucose concentration from an analyte monitoring device; receiving an alert condition set by a follower of the user at the follower's device; determining whether the alert condition is satisfied based on the information about the user's glucose concentration; and controlling the follower's device to output an alert when the alert condition is satisfied.

The information about the user's glucose concentration may be acquired using an analyte sensor at least partially inserted into the user's body.

The information about the user's glucose concentration may comprise a concentration value or a signal indicating the glucose concentration.

The alert condition may include a first condition comprising the user's glucose concentration being less than a first threshold value, a second condition comprising the user's glucose concentration being greater than a second threshold value that is greater than the first threshold value, and a third condition comprising a rate of change of the user's glucose concentration being greater than a third threshold value.

The alert condition may be transmitted from the follower's device to the electronic apparatus through a server.

The controlling may comprise transmitting alert information corresponding to the alert condition to the follower's device through the server.

Characteristics of alerts output by the follower's device may vary according to the alert condition, and when the alert is an alert sound, the characteristics may include pitch, intensity, and repetition cycle of the alert sound.

According to an embodiment of the present disclosure, there is provided a non-transitory computer-readable storage medium having stored thereon a program for executing the method of controlling the electronic apparatus on a computer device.

According to an embodiment of the present disclosure, there is provided an electronic apparatus comprising: a communication interface comprising at least one communication circuit; a memory storing at least one instruction; and at least one processor, wherein the at least one processor executes the at least one instruction to: receive, through the communication interface, information about a user's glucose concentration from an analyte monitoring device, receive, through the communication interface, an alert condition set by a follower of the user at the follower's device, determine whether the alert condition is satisfied based on the information about the user's glucose concentration, and control the follower's device to output an alert when the alert condition is satisfied.

Technical solutions of the present disclosure are not limited to the above-mentioned solutions, and solutions that are not mentioned will be clearly understood by those skilled in the art to which the present disclosure pertains from the present specification and the accompanying drawings.

### BRIEF DESCRIPTION OF DRAWINGS

Aspects, features, and advantages of specific embodiments of the present disclosure will become more apparent from the following description with reference to the accompanying drawings:
FIG. 1 is a schematic diagram illustrating a continuous glucose monitoring system according to an embodiment of the present disclosure.
FIG. 2 is a flowchart illustrating a method of providing alerts according to an embodiment of the present disclosure.
FIG. 3 is a screen showing an alert settings interface output by the second electronic apparatus according to an embodiment of the present disclosure.
FIG. 4 is a screen showing a detailed settings interface output by the second electronic apparatus according to an embodiment of the present disclosure.
FIG. 5 is a screen showing an alert interface output by the second electronic apparatus according to an embodiment of the present disclosure.
FIG. 6 shows a first filter screen (60) that is displayed when UI element (51) is selected.
FIG. 7 shows a second filter screen (70) that is displayed when UI element (52) is selected.
FIG. 8 is a block diagram illustrating the configuration of the analyte monitoring system (1000) according to an embodiment of the present disclosure.

### DESCRIPTION OF EXAMPLE EMBODIMENTS

After terms used in the present specification are briefly described, the present disclosure will be described in detail.

General terms that are currently widely used were selected as terms used in embodiments of the present disclosure in consideration of functions in the present disclosure, but may be changed according to the intention of those skilled in the art or a judicial precedent, the emergence of a new technique, and the like. In addition, in a specific case, terms arbitrarily chosen by an applicant may exist. In this case, the meaning of such terms will be mentioned in detail in a corresponding description portion of the present disclosure. Therefore, the terms used in the present disclosure should be defined on the basis of the meaning of the terms and the contents throughout the present disclosure rather than simple names of the terms.

The present disclosure may be variously modified and have several embodiments, and therefore specific embodiments of the present disclosure will be illustrated in the drawings and be described in detail in the detailed description. However, it is to be understood that the present disclosure is not limited to specific exemplary embodiments, but includes all modifications, equivalents, and substitutions without departing from the scope and spirit of the present disclosure. When it is determined that a detailed description of the known art related to the present disclosure may obscure the gist of the present disclosure, the detailed description will be omitted.

The terms "first," "second," and the like may be used to describe various components, but the components are not to be construed as being limited by these terms. The terms are used only to distinguish one component from another component.

Singular forms are intended to include plural forms unless the context clearly indicates otherwise. It should be understood that terms "include" or "comprise" used in the present specification, specify the presence of features, numerals, steps, operations, components, parts mentioned in the present specification, or combinations thereof, but do not preclude the presence or addition of one or more other features, numerals, steps, operations, components, parts, or combinations thereof.

Hereinafter, embodiments of the present disclosure will be described in detail with reference to the accompanying drawings so that those skilled in the art to which the present disclosure pertains may easily practice the present disclosure. However, the present disclosure may be modified in various different forms, and is not limited to embodiments described herein. In addition, in the drawings, portions unrelated to the description will be omitted to obviously describe the present disclosure, and similar reference numerals will be used to describe similar portions throughout the specification.

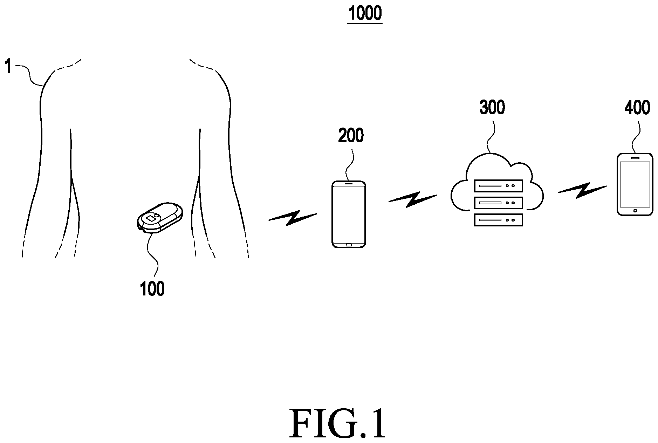

FIG. 1 is a schematic diagram illustrating a continuous glucose monitoring system according to an embodiment of the present disclosure.

Referring to FIG. 1, the analyte monitoring system 1000 may include an analyte monitoring device 100, a first electronic apparatus 200, a server 300, and a second electronic apparatus 400. The analyte monitoring system 1000 may be a Continuous Glucose Monitoring System (CGMS).

The analyte monitoring device 100 may measure an analyte signal related to the concentration of an analyte in a user's (1) body fluid. For example, the analyte may be glucose or ketone. The analyte monitoring device 100 may be wirelessly connected to the first electronic apparatus 200 and may transmit the analyte signal according to a predetermined period. For example, the analyte monitoring device 100 may transmit the analyte signal to the first electronic apparatus 200 every 1 minute or 5 minutes. The analyte monitoring device 100 may be attached to the user's (1) body, with a portion being inserted under the skin.

The analyte monitoring device 100 may store analyte signals for a predetermined period (e.g., 12 hours). Therefore, even if the connection between the analyte monitoring device 100 and the first electronic apparatus 200 is temporarily interrupted, as long as the interruption period does not exceed the predetermined period, the first electronic apparatus 200 can receive the stored analyte signals from the analyte monitoring device 100 after the connection is restored.

The first electronic apparatus 200 may provide information related to the analyte concentration to the user (1) based on the analyte signal received from the analyte monitoring device 100. For example, the first electronic apparatus 200 may display the user's (1) glucose concentration in real-time. The user (1) can monitor their glucose concentration in real-time through the first electronic apparatus 200. The first electronic apparatus 200 may include mobile devices such as smartphones, tablet PCs, or laptops that can communicate with the analyte monitoring device 100. Additionally, the first electronic apparatus 200 may include dedicated analyte data receivers or readers provided separately to the user (1). However, the first electronic apparatus 200 is not limited to these examples and may include any type of electronic device capable of communication and installation of programs or applications.

The communication link between the first electronic apparatus 200 and the analyte monitoring device 100 may be established through a glucose monitoring app running on the first electronic apparatus 200. The user (1) can input identification information (e.g., serial number or PIN code) of the analyte monitoring device 100 using the glucose monitoring app. For example, the user may scan a barcode on the package label of the analyte monitoring device 100 using the camera of the first electronic apparatus 200, or directly input it through an input interface such as a touchscreen. Once the identification information is entered, authentication between the first electronic apparatus 200 and the analyte monitoring device 100 may be performed based on this information. After successful authentication, the communication link between the two devices can be established. For example, the communication link between the first electronic apparatus 200 and the analyte monitoring device 100 may be established based on the Bluetooth protocol.

The server 300 may be connected to both the first electronic apparatus 200 and the second electronic apparatus 400, enabling it to transmit various information between these electronic devices (200, 400). For example, the server 300 may receive and store information about the user's (1) glucose concentration from the first electronic apparatus 200. The server 300 may transmit the stored glucose concentration information to the second electronic apparatus 400. Additionally, the server 300 may receive alert criteria set by the follower from the second electronic apparatus 400 and transmit them to the first electronic apparatus 200. The server 300 may be a cloud server or a physical server.

The second electronic apparatus 400 may provide information related to the analyte concentration to the user (1) or the user's (1) follower based on the data acquired by the first electronic apparatus 200. The second electronic apparatus 400 may receive data directly from the first electronic apparatus 200 or through the server. Unlike the first electronic apparatus 200, the second electronic apparatus 400 may not be able to directly transmit or receive signals from the analyte monitoring device 100).

The second electronic apparatus 400 may be a device of the user (1) or a follower. The follower, who monitors the user's (1) analyte concentration status, may be a healthcare provider such as a doctor or a guardian of the user (1). The second electronic apparatus 400 may be a smartphone, tablet PC, wearable device (e.g., smartwatch), analyte data receiver, or PC.

The second electronic apparatus 400 may receive information about the user's (1) glucose concentration from the server 300 and provide it to the follower. For example, the second electronic apparatus 400 may display a chart showing glucose concentration. Additionally, when there is an abnormality in glucose concentration, the second electronic apparatus 400 may output an alert. The second electronic apparatus 400 may receive alert conditions from the follower. The second electronic apparatus 400 may transmit these alert conditions to the first electronic apparatus 200 through the server 300.

Meanwhile, in the analyte monitoring system (1000), it is important to promptly notify followers when there is an abnormality in the user's glucose status. To detect abnormalities in the user's glucose levels as quickly as possible, it is effective for the user's device, which receives glucose information in real-time, to determine whether glucose conditions are met. This is because the server or external devices do not receive the user's glucose information directly from the analyte monitoring device, but rather receive it through the user's device. Therefore, in the analyte monitoring system (1000) according to this disclosure, there is an advantage that glucose abnormalities can be quickly detected by determining alert conditions at the user's device, and consequently, alerts can be promptly provided to both the user and followers.

In the analyte monitoring system (1000), followers can check the user's (1) biometric information in real-time. The biometric information may be the user's glucose information, which can include glucose values, event information (e.g., information about meals, exercise, insulin administration), and alerts related to glucose values. By allowing followers to check the user's biometric information in real-time, emergency situations can be prevented and the user's (1) psychological stability can be improved. For example, when a follower confirms that the user's real-time glucose information indicates hypoglycemia, they can take appropriate measures quickly, such as making an emergency call or administering medication to the user. In this way, medical efficiency can be increased as followers can remotely manage the user's (1) glucose status.

Hereinafter, for convenience of explanation, biometric information will be limited to glucose information, but it is not limited to this.

The second electronic apparatus 400 may acquire the user's (1) glucose information through a communication link with the first electronic apparatus 200). The communication link can be implemented in various ways. In one embodiment, the first electronic apparatus 200 and the second electronic apparatus 400 may be connected through an external system (e.g., server). In this case, the first electronic apparatus 200 transmits the user's (1) glucose information to the external system, and the external system can deliver it to the second electronic apparatus 400. The first electronic apparatus 200 and the second electronic apparatus 400 can be connected using the glucose monitoring app running on the first electronic apparatus 200 and the follower app running on the second electronic apparatus 400. Specifically, the first electronic apparatus 200 and the second electronic apparatus 400 can be connected based on accounts logged into each app.

In another embodiment, the first electronic apparatus 200 and the second electronic apparatus 400 can be directly connected without intervention from an external system. For example, the communication link can be created through Bluetooth, NFC, or Wi-Fi Direct. Meanwhile, the implementation method of the communication link can be determined based on the distance between the first electronic apparatus 200 and the second electronic apparatus 400 and the communication capabilities of each device.

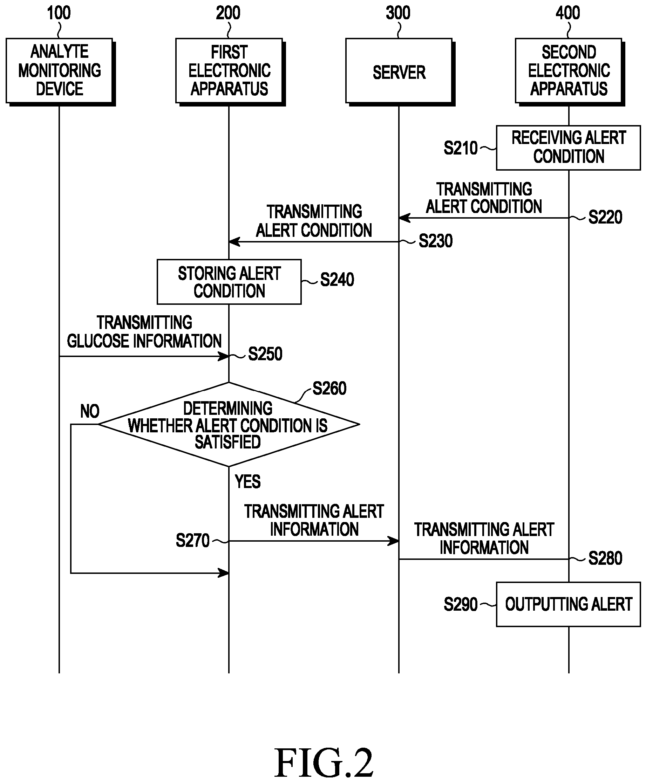

FIG. 2 is a flowchart illustrating a method of providing alerts according to an embodiment of the present disclosure.

Referring to FIG. 2, the second electronic apparatus 400 may receive alert conditions from a follower (S210). For example, the second electronic apparatus 400 may display UI elements for inputting alert conditions. The second electronic apparatus 400 may receive user input setting alert conditions through the UI elements. Additionally, the second electronic apparatus 400 may receive multiple alert conditions from the user.

The second electronic apparatus 400 may transmit the alert conditions to the server 300 (S220). The server 300 may transmit the alert conditions to the first electronic apparatus 200 (S230). The first electronic apparatus 200 may store the alert conditions (S240). In one embodiment, there may be multiple second electronic apparatuses 400 (i.e., multiple follower devices). In this case, the first electronic apparatus 200 may store multiple alert conditions corresponding to multiple follower devices. Each alert condition may be set at each follower device and transmitted to the first electronic apparatus 200 through the server 300. As will be described later, the second electronic apparatus 400 may determine whether alert conditions corresponding to each follower device are satisfied based on glucose information. Then, the second electronic apparatus 400 may provide alerts through the server 300 to follower devices corresponding to satisfied alert conditions. Accordingly, each follower can receive alerts based on their individually set alert conditions.

Alert conditions may include at least one condition related to glucose concentration. For example, alert conditions may include a first condition comprising glucose concentration being less than a first threshold value (hypoglycemia) and a second condition comprising glucose concentration being greater than a second threshold value (hyperglycemia), where the second threshold value may be set higher than the first threshold value. The first and second threshold values may be concentration values. Additionally, alert conditions may include a third condition comprising the rate of change of glucose concentration being greater than a third threshold value. The third threshold value may be a rate of change of concentration value over time.

The analyte monitoring device 100 may transmit glucose information to the first electronic apparatus 200 (S250). The glucose information, which relates to glucose concentration, may include signals (e.g., current or voltage) indicating concentration and concentration values. The analyte monitoring device 100 may transmit glucose information acquired in real-time to the first electronic apparatus 200. For example, the analyte monitoring device 100 may transmit glucose information every 1 minute or 5 minutes.

The first electronic apparatus 200 may determine whether alert conditions are satisfied based on the glucose information (S260). In one embodiment, the first electronic apparatus 200 may determine whether the glucose concentration is less than a first concentration (e.g., 70mg/dL). In another embodiment, the first electronic apparatus 200 may determine whether the glucose concentration is greater than a second concentration (e.g., 200mg/dL). In yet another embodiment, the first electronic apparatus 200 may determine whether the rate of change of glucose concentration is greater than a set value (e.g., 2mg/dL/min). Here, the first and second concentrations and the set value for glucose concentration rate of change may be values arbitrarily set by the user of the first electronic apparatus (e.g., patient) or the user of the second electronic apparatus (e.g., follower), or they may be fixed values pre-stored in the analyte monitoring device, but are not limited to these.

When multiple second electronic apparatuses 400 exist (i.e., multiple follower devices), multiple alert criteria set by each follower device may be stored in the first electronic apparatus 200. In this case, the second electronic apparatus 400 may determine whether alert criteria set by each follower device are satisfied based on the glucose information.

If it is determined that an alert condition is satisfied (S260: Yes), the first electronic apparatus 200 may transmit alert information to the server 300 (S270). The alert information may include information indicating the satisfaction of alert conditions, specifically information indicating which alert condition has been satisfied (including which follower device's alert condition has been satisfied). Additionally, alert information may include commands for outputting alerts corresponding to the satisfied alert conditions.

The server 300 may transmit the alert information to the second electronic apparatus 400 (S280). The second electronic apparatus 400 may receive the alert information from the server 300 (S280).

The second electronic apparatus 400 may output alerts based on the alert information (S290). The second electronic apparatus 400 may output alerts indicating that the user's glucose level is at a dangerous level. For example, the second electronic apparatus 400 may output a hypoglycemia alert when the user's glucose level is lower than a pre-set value, and may output a hyperglycemia alert when it is higher than a different pre-set value. Additionally, it may output a rapid glucose fluctuation alert when the rate of change of the user's glucose level is greater than a pre-set value. Here, the threshold values for hypoglycemia, hyperglycemia, and rapid glucose fluctuation may be directly set by the user or follower, but they may also be fixed values pre-stored in the analyte monitoring device. Moreover, as described later, each of these threshold values can be set differently. The alert may also include information about actions needed to improve glucose levels.

Alerts may have various types. For example, they may include vibration, sound, or text. Alert characteristics may vary depending on the alert conditions. When the alert type is sound, alert characteristics may include pitch, intensity, and repetition cycle of the sound. In one embodiment, alert characteristics may differ between when the alert condition is the first condition and when it is the second condition. Additionally, alert characteristics may vary depending on the glucose concentration or the level of risk indicated by the glucose concentration.

According to an embodiment of the present disclosure, the first electronic apparatus 200 may set and manage priorities of alert conditions. The alert conditions may have different priorities according to the level of risk to the user's health. For example, severe hypoglycemia conditions may have a first priority, hypoglycemia conditions may have a second priority, hyperglycemia conditions may have a third priority, and rapid glucose fluctuation conditions may have a fourth priority, where the first priority may be the highest and the fourth priority may be the lowest.

The first electronic apparatus 200 may apply different alert policies according to the priority of alert conditions. In one embodiment, the first electronic apparatus 200 may apply different transmission cycles for alerts according to the priority of alert conditions. For example, the first electronic apparatus 200 may transmit alerts at a first cycle (e.g., 1 minute) when severe hypoglycemia conditions are met, and at a second cycle (e.g., 5 minutes) when hypoglycemia conditions are met, where the first cycle may be shorter than the second cycle.

Additionally, the second electronic apparatus 400 may apply different alert output methods according to the priority of alert conditions. For example, even when the second electronic apparatus 400 is set to do-not-disturb mode, it may output alerts when first priority alert conditions (e.g., severe hypoglycemia) are met. In contrast, when alert conditions of third priority or lower are met, alerts may not be output in do-not-disturb mode.

The second electronic apparatus 400 may apply different alert sound characteristics according to the priority of alert conditions. In one embodiment, first priority alerts may have a first volume and a first repetition cycle, and second priority alerts may have a second volume and a second repetition cycle, where the first volume may be greater than the second volume and the first repetition cycle may be shorter than the second repetition cycle.

Furthermore, when high-priority alert conditions are met, the second electronic apparatus 400 may monitor whether the follower has acknowledged the alert. For example, if there is no acknowledgment from the follower for a predetermined time (e.g., 5 minutes) for a severe hypoglycemia alert, the second electronic apparatus 400 may gradually increase the alert volume, output alerts with vibration, or propagate the alert to other followers designated as emergency contacts.

According to an embodiment of the present disclosure, the first electronic apparatus 200 may classify and manage followers into multiple groups. For example, followers may be classified into medical staff group, family group, and general group. Each group may have different data access privileges and alert setting permissions.

The second electronic apparatus 400 of followers belonging to the medical staff group may access all of the user's glucose data and event information (e.g., records of meals, exercise, insulin administration). Additionally, the medical staff group may have administrator privileges to set or modify alert conditions for other follower groups. For example, medical staff can adjust alert thresholds for the family group based on the patient's condition.

The second electronic apparatus 400 of followers belonging to the family group may access real-time glucose data and some event information. The family group can set their own alert conditions but cannot modify alert conditions of other followers. For the general group, they can only check real-time glucose data, and their alert conditions may be set to predetermined default values.

In case of emergency situations, the first electronic apparatus 200 may propagate alerts gradually according to follower group priorities. For example, when severe hypoglycemia is detected, the first electronic apparatus 200 may first send alerts to the family group, and if there is no response within a predetermined time (e.g., 5 minutes), automatically send alerts to the medical staff group.

Additionally, the first electronic apparatus 200 may send differentiated alert content for each group. For example, it may send alerts containing detailed glucose levels and trend changes to the medical staff group, while sending alerts containing simple status information and recommended actions to the family group.

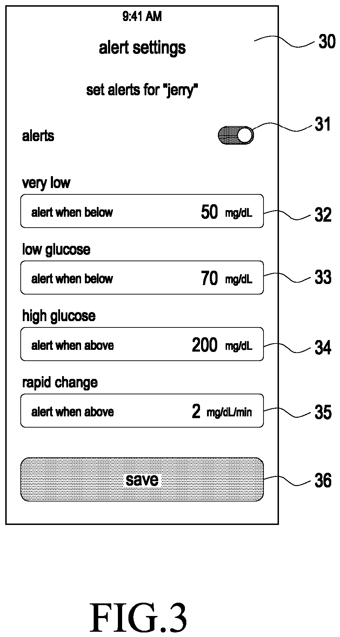

FIG. 3 is a screen showing an alert settings interface output by the second electronic apparatus according to an embodiment of the present disclosure.

Referring to FIG. 3, the alert settings screen 30 may include a first UI element 31 that can activate/deactivate alerts. For example, the first UI element 31 may be a switch. The alert settings screen 30 may include a second UI element 32 for inputting concentration values corresponding to severe hypoglycemia conditions, a third UI element 33 for inputting concentration values corresponding to hypoglycemia conditions, a fourth UI element 34 for inputting concentration values corresponding to hyperglycemia conditions, and a fifth UI element 35 for inputting concentration rate of change values corresponding to rapid glucose fluctuation conditions. The alert settings screen 30 may include a button 36 for saving alert settings.

Severe hypoglycemia refers to a state where the glucose value is below a threshold value that is lower than the hypoglycemia threshold value. Severe hypoglycemia may need to be set as a separate alert condition as it can be more critical to the patient than hypoglycemia. Additionally, rapid glucose fluctuation refers to an abnormally large change in glucose values (e.g., glucose spike). Since rapid glucose fluctuation can lead to risks of chronic diseases or cardiovascular diseases, it may need to be set as a separate alert condition.

Meanwhile, the method of providing alerts to followers may vary depending on the alert conditions. In one embodiment, the follower's device may output alerts with a first repetition cycle when severe hypoglycemia conditions are met, and with a second repetition cycle when hypoglycemia or hyperglycemia conditions are met. Here, the first repetition cycle may be shorter than the second repetition cycle. In another embodiment, when the follower's device is in do-not-disturb mode, alerts may be output when severe hypoglycemia conditions are met, but may not be output when hypoglycemia or hyperglycemia conditions are met.

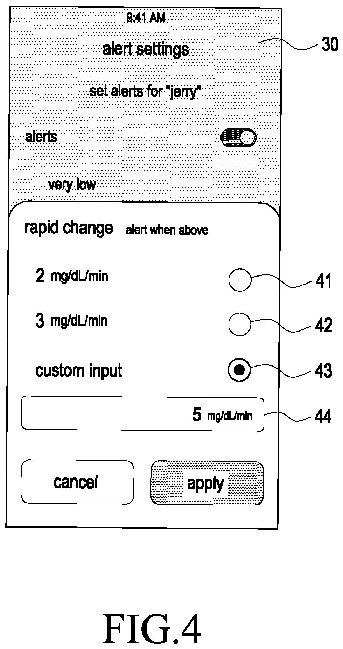

FIG. 4 is a screen showing a detailed settings interface output by the second electronic apparatus according to an embodiment of the present disclosure.

Referring to FIG. 4, the detailed settings screen 40 may be a screen for detailed configuration of values corresponding to conditions selected in the alert settings screen 30. For example, when the fifth UI element 35 is selected in the alert settings screen 30, a detailed settings screen 40 for rapid glucose fluctuation conditions may be displayed.

In one embodiment, the detailed settings screen 40 may be overlaid on the alert settings screen 30, covering at least a portion of the alert settings screen 30. In this case, the brightness of the uncovered portion of the alert settings screen 30 may be lower than the brightness of the detailed settings screen 40. This allows the follower to focus on the detailed settings screen 40. In another embodiment, the detailed settings screen 40 may be displayed by replacing, rather than covering, the alert settings screen 30.

The detailed settings screen 40 may include UI elements (41, 42) for selecting predetermined concentration rate of change values. The detailed settings screen 40 may include UI elements (43, 44) allowing followers to directly input concentration rate of change values. Specifically, when a follower selects UI element 43, LTI element 44 for inputting concentration rate of change values may be activated.

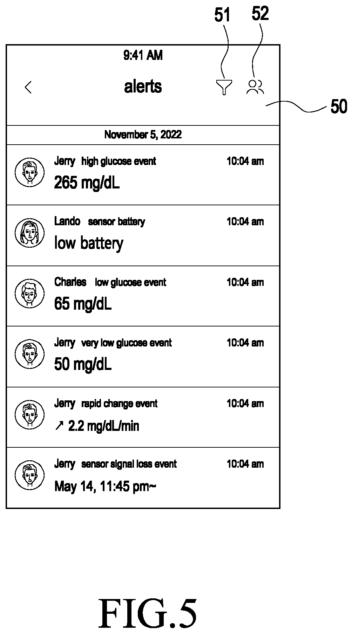

FIG. 5 is a screen showing an alert interface output by the second electronic apparatus according to an embodiment of the present disclosure.

Referring to FIG. 5, the alert screen 50 may include alert information related to glucose of people being followed by the follower who is a user of the second electronic apparatus 400. The second electronic apparatus 400 may be connected to multiple first electronic apparatuses 200 through the server 300 and may receive glucose-related alerts from these multiple first electronic apparatuses 200.

The alert screen 50 may include alert condition content, user profiles, glucose values, and alert occurrence times. In one embodiment, the alert screen 50 may include the first user's (Jerry) glucose value (265 mg/dL), the satisfied alert condition (hyperglycemia), and the alert occurrence time (10:04 AM). In another embodiment, the alert screen 50 may include the second user's (Charles) glucose value (65 mg/dL), the satisfied alert condition (hypoglycemia), and the alert occurrence time (10:04 AM).

Among the alerts provided to followers, there may also be alerts unrelated to the user's glucose. For example, these may include alerts notifying low battery of the user's sensor or alerts notifying loss of glucose signal from the user.

The alert screen 50 may include a UI element 51 for filtering displayed alert types and a UI element 52 for filtering users being followed by the follower.

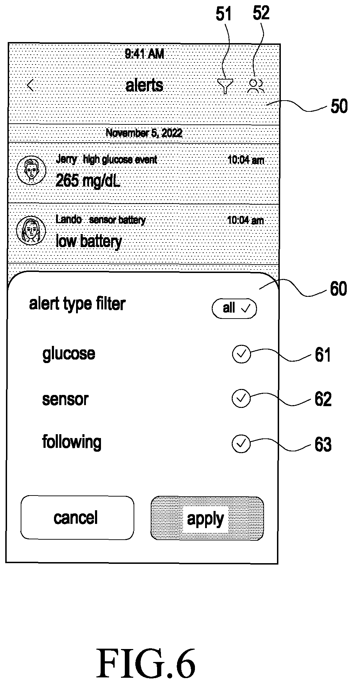

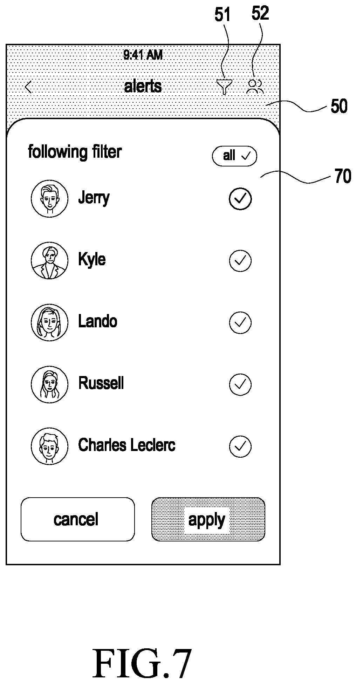

FIG. 6 shows a first filter screen that is displayed when UI element 51 is selected. FIG. 7 shows a second filter screen that is displayed when UI element 52 is selected.

Referring to FIG. 6, the first filter screen 60 may include a UI element 61 for selecting first type alerts related to the user's glucose, a UI element 62 for selecting second type alerts related to the user's sensor, and a UI element 63 for selecting third type alerts related to users being followed by the follower. The follower can set necessary types of alerts by selecting UI elements 61, 62, and 63.

Referring to FIG. 7, the second filter screen 70 may include a list of multiple users being followed by the follower. The follower can select users from whom they want to receive alerts from the list. Meanwhile, the second filter screen 70 may be displayed when the follower selects either UI element 52 in FIG. 5 or UI element 63 in FIG. 6.

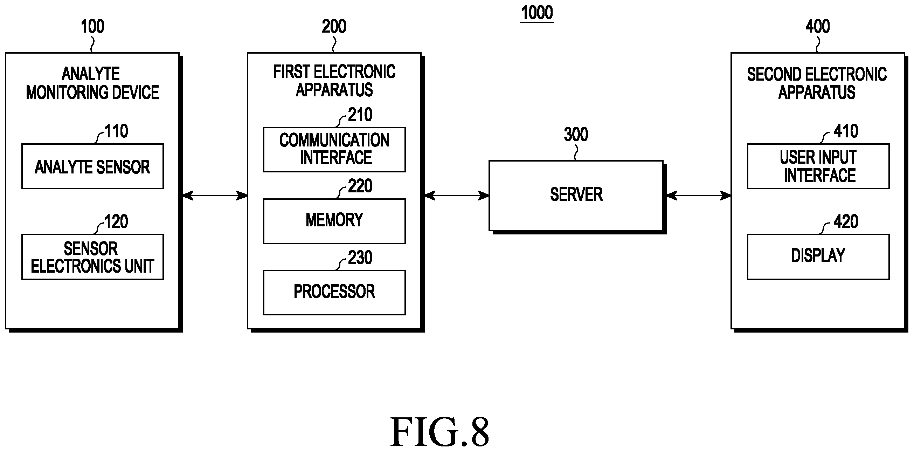

FIG. 8 is a block diagram illustrating the configuration of the analyte monitoring system 1000 according to an embodiment of the present disclosure.

Referring to FIG. 8, the analyte monitoring device 100 may include an analyte sensor 110 and a sensor electronics unit 120. The analyte sensor 110 may be a component for sensing analyte signals (or sensor signals). The analyte sensor 110 may include a sensor probe that is at least partially inserted into the body. A sensing region that reacts with glucose in the body to measure blood glucose may be formed on the sensor probe.

The sensor electronics unit 120 may transmit sensor signals acquired through the analyte sensor 110 or concentration values derived from the sensor signals to the first electronic apparatus 200. The sensor electronics unit 120 may transmit sensor signals or concentration values to the first electronic apparatus 200 at predetermined intervals (e.g., 1 minute). The sensor electronics unit 120 may include at least one communication interface. The sensor electronics unit 120 may perform communication with the first electronic apparatus 200 through the communication interface. For example, the communication interface may include Bluetooth modules, low-power Bluetooth modules, RF modules, and NFC modules.

Additionally, the sensor electronics unit 120 may include an operating system (OS) for controlling the overall operation of the components of the analyte monitoring device 100, and a memory storing instructions or data related to the components of the analyte monitoring device 100. The sensor electronics unit 120 may include a processor that is electrically connected to the memory and controls the overall functions and operations of the analyte monitoring device 100.

The first electronic apparatus 200 may include a communication interface 210, a memory 220, and a processor 230. Additionally, the first electronic apparatus 200 may include a display for displaying the user's glucose values.

The communication interface 210 may include at least one communication circuit. The communication interface 210 may receive sensor signals or concentration values from the analyte monitoring device 100.

The memory 220 may store an operating system (OS) for controlling the overall operation of the components of the first electronic apparatus 200 and instructions or data related to the components of the first electronic apparatus 200. The memory 220 may be implemented as non-volatile memory (e.g., hard disk, SSD (Solid State Drive), flash memory) or volatile memory. The memory 220 may store alert conditions set by followers at the second electronic apparatus 400. The memory 220 may store multiple alert conditions set by multiple second electronic apparatuses 400.

The processor 230 may be electrically connected to the memory 220 and control the overall functions and operations of the first electronic apparatus 200. The processor 230 may control the first electronic apparatus 200 by executing instructions stored in the memory 220.

The processor 230 may receive information about the user's glucose concentration from the analyte monitoring device 100 through the communication interface 210. The information about glucose concentration may be concentration values or sensor signals indicating glucose concentration. The information about glucose concentration may be acquired using the analyte sensor 110.

The processor 230 may receive alert conditions set by users (i.e., followers) of the second electronic apparatus 400 through the communication interface 210. The alert conditions may include a first condition comprising the user's glucose concentration being less than a first threshold value, a second condition comprising the user's glucose concentration being greater than a second threshold value, and a third condition comprising a rate of change of the user's glucose concentration being greater than a third threshold value. Here, the second threshold value may be set greater than the first threshold value, but is not limited to this. The multiple alert conditions may be transmitted from the second electronic apparatus 400 to the first electronic apparatus 200 through the server 300.

The processor 230 may determine whether alert conditions are satisfied based on information about the user's glucose concentration. When alert conditions are satisfied, the processor 230 may control the second electronic apparatus 400 to output alerts. The processor 230 may transmit alert information corresponding to alert conditions to the second electronic apparatus 400 through the server 300. Characteristics of alerts output by the second electronic apparatus 400 may vary according to alert conditions. When alerts are output as sound, alert characteristics may include pitch, intensity, and repetition cycle of the alert sound.

The processor 230 may control the display (not shown) to display the user's glucose values.

According to an embodiment of the present disclosure, the first electronic apparatus 200 may operate adaptively according to network conditions. The first electronic apparatus 200 may periodically monitor the communication status with the server 300 and ensure that alerts are not delayed or missed even when the communication status is unstable or disconnected.

For example, when communication with the server 300 is disconnected, the first electronic apparatus 200 may store events where alert conditions are met in the memory 220. When communication is restored, the first electronic apparatus 200 may check the timestamps of the stored events and only transmit alerts for events that occurred within a predetermined time period (e.g., 30 minutes). This can prevent multiple outdated alerts from being sent simultaneously when communication is restored.

Additionally, when the communication status with the server 300 is unstable, the first electronic apparatus 200 may adjust the transmission frequency or data size of alert data. For example, when network bandwidth is limited, the first electronic apparatus 200 may adjust the sampling period of glucose data or selectively transmit only high-priority alerts.

When communication with the server 300 is completely disconnected, the first electronic apparatus 200 may attempt to switch to direct communication with the second electronic apparatus 400. For example, when the first electronic apparatus 200 and the second electronic apparatus 400 are on the same local network, direct communication can be established through Bluetooth or Wi-Fi Direct. In this case, urgent alerts such as severe hypoglycemia can be transmitted directly without going through the server 300.

The first electronic apparatus 200 may attempt to retransmit when alert transmission fails. During retransmission, the number and interval of retransmission attempts can be adjusted based on the alert priority and elapsed time. For example, for severe hypoglycemia alerts, more frequent retransmission attempts may be made at shorter intervals, while for general alerts, fewer retransmission attempts may be made at relatively longer intervals.

According to an embodiment of the present disclosure, the first electronic apparatus 200 may efficiently determine whether alert conditions are satisfied while optimizing battery consumption. The processor 230 may apply different processing cycles and methods depending on the characteristics of alert conditions.

For example, the processor 230 may apply different determination cycles depending on the type of alert condition. For rapid glucose fluctuation conditions, which require calculating the rate of change between current and previous glucose values, determination may be performed every time data is received. In contrast, for hyperglycemia or hypoglycemia conditions, which only require comparing the current glucose value with a threshold, the determination cycle can be variably applied depending on the degree of glucose value changes.

The processor 230 may adjust processing methods according to remaining battery level. When the battery level is below a first threshold (e.g., 20%), the processor 230 may switch to low-power mode to perform alert condition determinations. In low-power mode, determinations may be performed only for high-priority alert conditions (e.g., severe hypoglycemia) or the determination cycle may be increased. However, even when the battery level is critically low, below a second threshold (e.g., 5%), detection of emergency situations such as severe hypoglycemia can still be performed normally.

Additionally, the processor 230 may optimize battery usage by learning the user's glucose patterns. For example, during time periods when the user's glucose levels remain stable, the alert condition determination cycle can be increased, while during periods of high variation (e.g., before and after meals), the determination cycle can be decreased. This allows for reduced battery consumption while not missing important events.

The first electronic apparatus 200 may also perform battery optimization when transmitting alert information. For example, when transmitting data to the server 300, it may reduce the number of transmissions through batch processing or adjust transmission power according to network signal strength. However, urgent alerts may be transmitted immediately without batch processing.

The second electronic apparatus 400 may include a user input interface 410, a display 420, a second communication interface 430, a second memory 440, and a second processor 450. For example, the second electronic apparatus 400 may be a smartphone. The user input interface 410 receive alert conditions from followers. For example, the user input interface 410 may be a touch screen. The display 420 may display alerts about the user of the first electronic apparatus 200. For example, the display 420 may display alert messages indicating the user's glucose status.

The second electronic apparatus 400 may include a communication interface comprising at least one communication circuit. The communication interface may transmit alert conditions to the server 300. The communication interface may receive alert information from the server 300. The second electronic apparatus 400 may include a memory storing an operating system (OS) for controlling the overall operation of the components of the second electronic apparatus 400 and instructions or data related to the components of the second electronic apparatus 400. The second electronic apparatus 400 may include a processor that is electrically connected to the memory and controls the overall functions and operations of the second electronic apparatus 400.

Various exemplary embodiments of the present disclosure described above may be implemented in a computer or a computer readable recording medium using software, hardware, or a combination of software and hardware. In some cases, embodiments described in the present disclosure may be implemented by the processor itself. According to a software implementation, embodiments such as procedures and functions described in the present disclosure may be implemented by separate software modules. Each of the software modules may perform one or more functions and operations described in the present disclosure.

Computer instructions for performing processing operations according to the diverse embodiments of the present disclosure described above may be stored in a non-transitory computer-readable medium. The computer instructions stored in the non-transitory computer-readable medium allow a specific machine to perform the processing operations according to the diverse embodiments described above when they are executed by a processor.

The non-transitory computer-readable medium is not a medium that stores data for a while, such as a register, a cache, a memory, or the like, but means a medium that semi-permanently stores data and is readable by the apparatus. A specific example of the non-transitory computer-readable medium may include a compact disk (CD), a digital versatile disk (DVD), a hard disk, a Blu-ray disk, a universal serial bus (USB), a memory card, a read only memory (ROM), or the like.

The machine-readable storage medium may be provided in the form of a non-transitory storage medium. Here, the "non-transitory storage medium" means that the storage medium is a tangible device, and does not include a signal (for example, electromagnetic waves), and the term does not distinguish between the case where data is stored semi-permanently on a storage medium and the case where data is temporarily stored thereon. For example, the "non-transitory storage medium" may include a buffer in which data is temporarily stored.

The methods according to the diverse embodiments disclosed in the document may be included and provided in a computer program product. The computer program product may be traded as a product between a seller and a purchaser. The computer program product may be distributed in the form of a machine-readable storage medium (for example, compact disc read only memory (CD-ROM)), or may be distributed (for example, download or upload) through an application store (for example, Play Store^{™}) or may be directly distributed (for example, download or upload) between two user devices (for example, smart phones) online. In a case of the online distribution, at least some of the computer program products (for example, downloadable app) may be at least temporarily stored in a machine-readable storage medium such as a memory of a server of a manufacturer, a server of an application store, or a relay server or be temporarily created.

According to an embodiment of the present disclosure, alert conditions set by followers can be promptly evaluated and alerts can be provided to followers accordingly. This enables followers to take measures to prevent users from entering dangerous situations.

In addition, effects obtainable or predicted by the embodiments of the present disclosure have been disclosed directly or implicitly in the detailed description of the embodiments of the present disclosure. For example, various effects predicted according to embodiments of the present disclosure have been disclosed in the above-described detailed description.

Other aspects, advantages and prominent features of the present disclosure will become apparent to those skilled in the art from the above detailed description which disclosed various embodiments of the present disclosure taken in conjunction with the accompanying drawings.

Although embodiments of the present disclosure have been illustrated and described hereinabove, the present disclosure is not limited to the above-described specific embodiments, but may be variously modified by those skilled in the art to which the present disclosure pertains without departing from the gist of the present disclosure as disclosed in the accompanying claims. These modifications should also be understood to fall within the scope and spirit of the present disclosure.

## Claims

1. A method performed by an electronic apparatus, the method comprising:
receiving information about a user's glucose concentration from an analyte monitoring device attached to the user's body;
receiving an alert condition set by a follower of the user at the follower's device;
determining whether the alert condition is satisfied based on the information about the user's glucose concentration; and
controlling the follower's device to output an alert when the alert condition is satisfied.

2. The method of claim 1, wherein
the information about the user's glucose concentration is acquired using an analyte sensor at least partially inserted into the user's body.

3. The method of claim 1, wherein
the information about the user's glucose concentration comprises a concentration value or a signal indicating the glucose concentration.

4. The method of claim 1, wherein
the alert condition includes at least one of
a first condition comprising the user's glucose concentration being less than a first threshold value,
a second condition comprising the user's glucose concentration being greater than a second threshold value, and
a third condition comprising a rate of change of the user's glucose concentration being greater than a third threshold value,
wherein the second threshold value is greater than the first threshold value.

5. The method of claim 1, wherein
the alert condition is transmitted from the follower's device to the electronic apparatus through a server.

6. The method of claim 1, wherein
the controlling comprises:
transmitting alert information corresponding to the alert condition to the follower's device through a server.

7. The method of claim 1, wherein
characteristics of the alert output by the follower's device vary according to the alert condition, and
wherein the characteristics comprise pitch, intensity, and repetition cycle when the alert is an alert sound.

8. A non-transitory computer-readable storage medium storing instructions that, when executed by at least one processor, cause the at least one processor to perform the method of claim 1.

9. An electronic apparatus comprising:
a communication interface comprising at least one communication circuit;
a memory storing at least one instruction; and
at least one processor,
wherein the at least one processor is configured to execute the at least one instruction to:
receive, through the communication interface, information about a user's glucose concentration from an analyte monitoring device attached to the user's body,
receive, through the communication interface, an alert condition set by a follower of the user at the follower's device,
determine whether the alert condition is satisfied based on the information about the user's glucose concentration, and
control the follower's device to output an alert when the alert condition is satisfied.

10. The electronic apparatus of claim 9, wherein
the information about the user's glucose concentration is acquired using an analyte sensor at least partially inserted into the user's body.

11. The electronic apparatus of claim 9, wherein
the information about the user's glucose concentration comprises a concentration value or a signal indicating the glucose concentration.

12. The electronic apparatus of claim 9, wherein
the alert condition includes at least one of
a first condition comprising the user's glucose concentration being less than a first threshold value,
a second condition comprising the user's glucose concentration being greater than a second threshold value, and
a third condition comprising a rate of change of the user's glucose concentration being greater than a third threshold value,
wherein the second threshold value is greater than the first threshold value.

13. The electronic apparatus of claim 9, wherein
the alert condition is transmitted from the follower's device to the electronic apparatus through a server.

14. The electronic apparatus of claim 9, wherein
the at least one processor is further configured to execute the at least one instruction to transmit alert information corresponding to the alert condition to the follower's device through a server.

15. The electronic apparatus of claim 9, wherein
characteristics of the alert output by the follower's device vary according to the alert condition, and
wherein the characteristics comprise pitch, intensity, and repetition cycle when the alert is an alert sound.
